# EUROPEAN PATENT APPLICATION

(11) **EP 3 725 337 A1**
(43) Date of publication of application: **21.10.2020**
(21) Application number: 19020294.5
(22) Date of filing: 18.04.2019
(51) Int. Cl.: A61L 2/20, C02F 1/78, A61L 2/24

(54) **CONTROL DEVICE AND CONTROL METHOD FOR LIQUID DISPENSERS AND LIQUID PURIFIERS**

(71) Applicant: La Fantana Srl, Bucharest (RO)
(72) Inventor: Amza, Cristian, Bucharest (RO)
(74) Representative: Cosmovici, Paul

(57) **Abstract**

Disclosed herein is a control device for a liquid dispenser or liquid purifier, wherein the control device comprises: a communication unit configured to receive data from a remote computing device, and a control unit configured to control an ozone-based liquid cleaning operation in the liquid dispenser or liquid purifier based on the received data.

Further disclosed herein is a computer-implemented control method for a liquid dispenser or liquid purifier, wherein the method comprises: receiving data from a remote computing device, and controlling an ozone-based liquid cleaning operation in the liquid dispenser or liquid purifier based on the received data.

## Description

### Technical field

This invention refers to a control device and a computer-implemented method for controlling an ozone-based liquid cleaning operation in a liquid dispenser or liquid purifier. In particular, the invention may refer to an equipment for the control of water ozonation and for the management and communication with water dispensers and purifiers installed in different locations, meant to be used for water dispenser type devices and water purifiers type devices.

### Background

Ozone is a strong oxidant and disinfectant, with clear qualities in improving the taste, smell and color of water. In Nice, France, ozone is used to disinfect water for the first time worldwide, starting with the year 1906.

Ozone is a colorless gas, being the second one in the list of the most powerful oxidants, after fluorine. It exercises its antimicrobial effect in a span of 1-2 seconds, does not release secondary chemical compounds as chlorine does, and maintains water clean, clear and fresh. Furthermore, ozone may be used to clean or purify various other liquids than water.

It is obtained, e.g., through the passing of air or oxygen between two electrodes with a potential difference. After being formed, it is introduced in the water where, after acting as a disinfectant, it decomposes into oxygen, without negatively affecting the organoleptic and physico-chemical characteristics of water.

Currently, there are known therapeutic ozonators as the one in the document RO 122183 that is used for medical purposes and is composed of a high voltage source, an ozone generator which is supplied through an air pump or with bottled oxygen in a container, the generator releasing the ozone through a corrosion-resistant hose and directing it into a surface applications nozzle or in a porous disperser that disperses the ozone in fine bubbles in a container that can contain various liquids.

### Summary

The technical problem that the present invention settles refers to how to allow remote management of and communication with liquid dispensers or purifiers and, in particular, in the remote control of the process of ozonating liquids, such as water or drinking water, provided by the liquid dispenser or purifier, as well as the remote update and viewing of the data communicated.

Aspects of the present invention relate to a control device according to claim 1, a liquid cleaning device according to claim 7, a liquid cleaning system according to any one of claims 10 and 13, a multiple-location liquid cleaning system according to claim 14 and a computer-implemented control method according to claim 15. Claims 2 to 6 refer to specifically advantageous realizations of the control device according to claim 1; claims 8 and 9 refer to specifically advantageous realizations of the liquid cleaning device according to claim 7; and claims 11 and 12 refer to specifically advantageous realizations of the liquid cleaning system according to claim 10.

When referring in this application to a liquid, it shall be understood that this may, for example, be water or drinking water; however, the present invention is not restricted to water and may be applied to various other liquids as well. Correspondingly, as used herein, a water dispenser or purifier is an example of a liquid dispenser or purifier, a water canister is an example of a liquid canister, a water supply is an example of a liquid supply, a water cleaning device is an example of a liquid cleaning device, a water cleaning system is an example of a liquid cleaning system, and a water cleaning operation is an example of a liquid cleaning operation.

Throughout this application, a control unit, which is a part of the control device that receives data from a remote computing device, may be realized as a microcontroller.

As used herein, a communication unit, which is a part of the control device that receives data from the remote computing device, may be realized as a communication system and may be based on Global System for Mobile Communications, GSM, and/or on General Packet Radio Service, GPRS.

In the present disclosure, a water cleaning unit, which performs the water cleaning operation in a water dispenser or water purifier may, for example, be an ozonator.

When referring herein to an ozone-based water cleaning operation, it shall be understood that this may, in particular, be an ozonation process.

Throughout this application, the remote computing device may be realized as a server or a mobile terminal device.

As used herein, data received from the remote computing device may comprise an ozonation program for controlling the ozone-based water cleaning operation and/or an operation program for controlling the water cooler and heater of a water dispenser.

In the present disclosure, a user interface may be a management solution or a web interface or an Android app; moreover, an app for a mobile computing device may be an Android app.

When referring herein to a user of the control device, it shall be understood that this user may be a client or buyer of the control device; on the other hand, a user of the user interface may be a provider or seller of the control device.

Throughout this application, a system which comprises a water cleaning device, an external computing device, a user interface and a switch for counting canisters on a water dispenser, and which is used for a water dispenser, may be referred to as an equipment for the control of the water ozonation and management and communication with water dispensers installed in various locations. Similarly, a system which comprises a water cleaning device, an external computing device, a user interface and a valve (e.g. solenoid valve) for controlling a water supply of a water purifier, and which is used for a water purifier, may be referred to as an equipment for the control of the water ozonation and management and communication with water purifiers installed in various locations.

The control device and control method according to the present invention, or the equipment for controlling the water ozonation process and for managing and communicating with the water dispensers and purifiers installed in different locations according to the present invention, solve the above-mentioned technical problem. When the equipment is used for water dispensers, it may be composed of an ozone generator, an air pump, which retrieves air from the atmosphere and pumps it through a tube into the ozone generator, a switch with the purpose of a canister counter which is installed within or at the dispenser and placed with the actuator on the contact surface between the canister and the dispenser. Furthermore, it may be equipped with a GSM communication system, an internal clock equipped with an internal backup battery, hardware and software protections, a server, a web interface and an Android app. Some or all of these components may be based on or connected with or controlled by a microcontroller.

When the equipment is used for purifier-type devices it may be composed of an ozonator, a valve (e.g. a solenoid valve), a GSM communication system, an internal clock, hardware and software protections, a server, a web interface and an Android app.

According to the invention, the advantages of a control device and control method or of an equipment for the control of water ozonation and for managing and communicating with water dispensers and purifiers installed in different locationsinclude: First, the ozonation program may be developed following a preset program or may be a preset program; the ozonation program may be received from a remote computing device and may be stored in a memory of the control device. The ozonation program may be a program for controlling the ozone-based water cleaning operation. Second, the operation program can be set in an energy-saving manner or can set the water dispenser or purifier in an energy-saving mode; the operation program may be a program for controlling a heater and cooler of a water dispenser. The heater and cooler of the water dispenser may have the function of heating or cooling the drinking water stored in a canister or in a tank of the water dispenser. Third, a GSM communication with the server is set up; thereby, the control device may receive data from the server and may send data to the server. Fourth, one can remotely stop the energy supply of the dispenser or the water supply of the purifier. Fifth, the program can be set or updated remotely and may be sent to the control device via the communication unit of the control device.

In other words, the claimed control device and control method allow to remotely control the ozone-based water cleaning operation executed at a water purifier or water dispenser. Moreover, the claimed control device and control method allow for control of an easily scalable number of multiple water purifiers or water dispensers, which can even be located at different locations.

### Brief description of the drawings

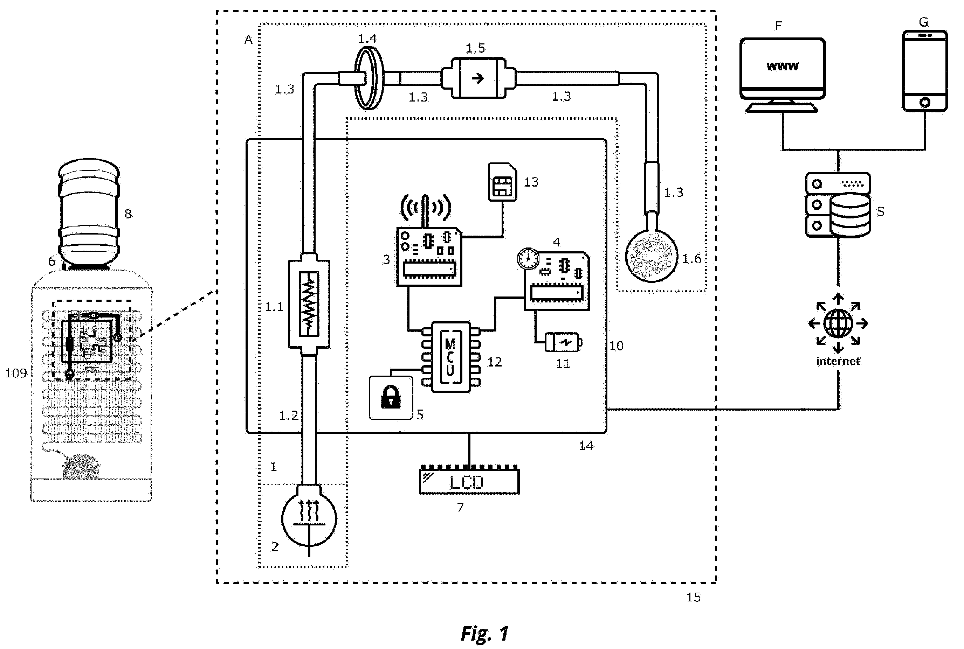

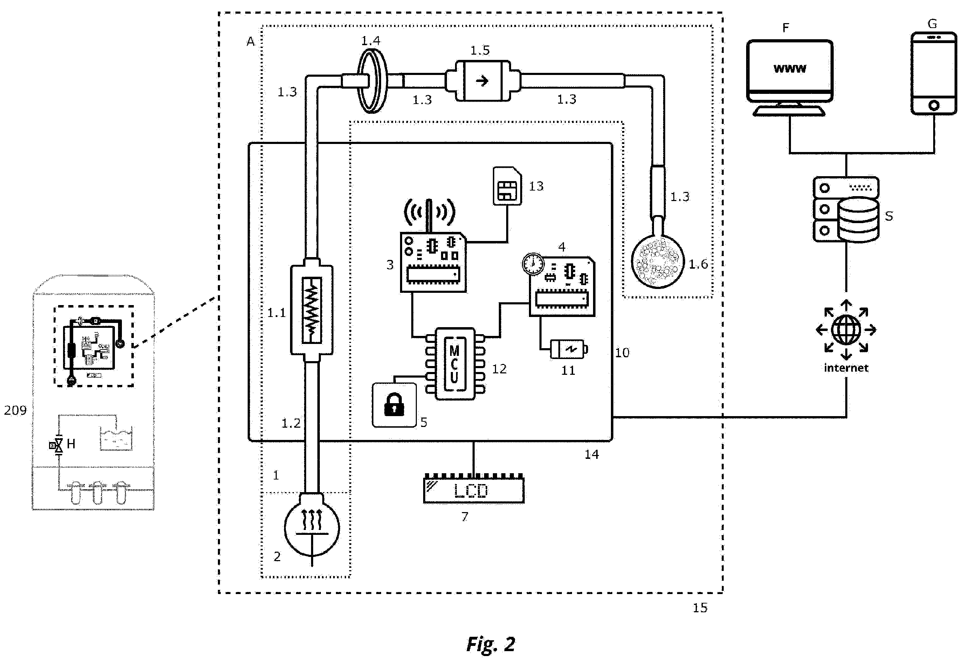

Next, we are presenting two options of the control device / water cleaning device / water cleaning system / multiple-location water cleaning system / equipment for the water ozonation control and managing and communicating with the water dispensers and purifiers installed in different locations, according to figures 1 or 2. Fig. 1 illustrates a multiple-location water cleaning system comprising a water dispenser, thus corresponding to the first variant of the present invention described below. Fig 2 illustrates a multiple-location water cleaning system comprising a water purifier, thus corresponding to the second variant of the present invention described below.

### Detailed description

Next, we present two exemplary variants of the control device / water cleaning device / water cleaning system / multiple-location water cleaning system / equipment for the water ozonation control and managing and communicating with water dispensers or purifiers installed in different locations, corresponding to Fig. 1 or Fig. 2, respectively. The first variant refers to a water dispenser 109, and the second variant refers to a water purifier 209.

### First variant

A water cleaning system in the first variant, as illustrated in Fig. 1, comprises one or multiple water cleaning systems and a remote computing device S, wherein each of the water cleaning systems in the first variant comprises a water dispenser 109, and wherein the water dispensers may be installed at different locations.

The water cleaning system in the first construction variant comprises a water cleaning device 15 and a water dispenser 109. Attached at, such as on or in the water dispenser 109 may be a water canister 8 or a plurality of water canisters 8.

Furthermore, the water cleaning system may comprise a switch 6 with the purpose of a canisters counter.

The water cleaning device 15 comprises a water cleaning unit A, such as an ozonator A, and a control device 14.

The control device 14 comprises a control unit 12, such as a microcontroller 12, and a communication unit 3, such as a GSM communication system 3, which may use a SIM card 13. Furthermore, the control device 14 may comprise an internal clock 4, which may be equipped with an internal backup battery 11, hardware and/or software protections 5. and/or a display 7.

The communication unit 3 is configured to receive data from a remote computing device S, such as a server S or a mobile terminal device S. Furthermore, the communication unit 3 may be configured to send data to the remote computing device S. The data sent to the remote computing device S may be output in a user interface, wherein the data received from the remote computing device S may be based on a user input in the user interface, and wherein the user interface comprises at least one of a web interface F and an app G for a mobile computing device, such as an Android app G.

The equipment for the control of water ozonation and management and communication with water dispensers installed in different locations may be based on a control unit 12, such as a microcontroller 12, with flash memory, EEPROM (non-volatile memory) and an external EEPROM memory (non-volatile memory).

The water cleaning unit A, such as the ozonator A, comprises an ozone generator 1 and an air pump 2.

With the help of the air pump 2, the air from the atmosphere is retrieved and pumped through a silicone tube into the ozone generator 1. After the completion of the ozone-based water cleaning operation, such as the ozonation process, the pump 2 may work for another time interval, such as for another few minutes, in order to allow ozone to decompose into oxygen.

The ozone generator 1 is an electronic device which may comprise an enclosure 1.1 connected to an inlet tube 1.2 and an outlet tube 1.3. The inlet tube 1.2 is connected to the air pump 2, receiving air with oxygen from the atmosphere, while through the outlet tube 1.3 after the ozone production process, air is released with ozone. The process itself may be based on converting air in ozone through electrical discharges, using high voltages (corona discharge).

The ozonation process may function daily following a prescheduled program, that can last 10-15 minutes, and which can be configured through the web interface F and communicated to the equipment through the GSM/GPRS system 3.

The ozone generator 1 may further comprise an air filter 1.4, which does not allow particles in the air being larger than 0.45 micrometers or larger than another predetermined size to get into the water basin or tank. In order to prevent the water in the water basin or tank from getting into the ozone generator 1, it may be equipped with a check valve 1.5, and in order to maximize the bubble generation surface, a spherical diffusion stone 1.6 may be used, wherein the stone may be made of corundum. During the period in which the ozonation process takes place water consumption may be not recommended; this period is, however, configurable with the use of a web interface F and may be visually signaled on a display 7 or through a light-emitting diode, LED, which, for example, may turn red.

The switch 6 may be an electromechanical switch that helps detect the presence of a water canister 8 on the water dispenser 9. The switch 6 may be installed within or at the dispenser 9 and positioned with the switch actuator on the contact surface between the canister 8 and the dispenser 9. The switch 6 may be usually set on *open* when no canister is present on the dispenser, and set *closed* when there is a canister present on the dispenser. By detecting the presence or absence of the water canister 8, one can keep count of the number of canisters that have been replaced/changed on that dispenser, by updating an index. The index represents the total count of the number of canisters changed and it is stored locally, on the equipment, for example in a non-volatile memory.

The GSM communication system 3 installed on the equipment for the control of water ozonation and the management of and communication with water dispensers and purifiers installed in different locations according to the invention may contain a GSM/GPRS (2G), quad-band modem which uses a SIM card for the bidirectional data exchange with the server S.

The communication between the equipment and the server S may be performed through standard Internet protocols (socket TCP), where the equipment may be a TCP client which connects to the TCP server, identified through IP/name and port.

The equipment can be configured locally, through a serial interface, for setting the GSM/GPRS configuration parameters of the ozonation and operation program.

Also, through this serial interface, the equipment can be locally diagnosed. The GSM module is used for notifying any equipment errors for the following components: the air pump 2, the ozone generator 1, the internal clock 4, or in order to detect the cause of the last restart of the equipment.

Through the GSM communication system 3, the hour and date can be updated remotely.

The electrical supply of the equipment may be performed using a 230VAC network. The equipment may include AC-DC and DC-DC converters, used for powering internal components.

The equipment may also include an electrical output of 230VAC/10A, controlled by software through a relay.

The electrical supply of the water dispenser 9, namely the cooler and the heater, may be provided through the electric output which is controlled by the equipment. The operation program of the water cooler and heater may be configurable from the web interface F for each day of the week.

The controlled stop function can be also used permanently for deadbeat clients or in case of a payment default. In this case, for dispensers with canisters, stopping the supply will no longer allow the cooling or heating of the water, and for equipment supplied from the network, the controlled stop will completely stop the water supply.

The equipment may also be equipped with an internal clock 4 which indicates the current hour/date, including the day of the week, being also supplied with an internal backup battery 11. In this way, when the main electricity supply is interrupted in case of any blackout, the set hour/date is maintained. The equipment may automatically memorize a timestamp for the first and last switch from the main supply to the internal battery (the first and the last blackout).

The hardware and software protections 5 of the equipment may be arranged in a housing 10 made of spill-proof plastic material and consist in a safety fuse, used in case of overcurrent, in a special watchdog timer, WDT timer, used to return from difficult SW situations, in a SW timer for limiting the maximum ozonation period, or in a checksum for protecting the data stored and those sent through GSM/GPRS.

Housing 10 may also hold the microcontroller 12.

Server S may perform the communication between the equipment and a back-office. Each equipment may be identified by the modem's IMEI (the unique identifier of the equipment). Communication may be made based on a package containing all the settings, update information, errors, etc.

Communication with the server may be performed by the equipment as follows: when detecting a new canister; or within a timeframe pre-defined from the web interface (advisable between 1-4 hours). Unless the equipment opens communications with the server, no modifications can be communicated to it. Communication is made through GPS/GPRS.

The web interface F enables the interaction of users with the equipment. In the interface one can configure equipment at company, location or individual level. The information managed on the equipment from the web may comprise one or more of the following: identity information of a user or client of the control device; identity information of the control device, such as the International Mobile Equipment Identity, IMEI; location information, such as location information of the water dispenser or water purifier; status of the water dispenser (such as: has / does not have a canister); status of the water cleaning operation or of the control device (such as: water cleaning operation is running / water cleaning operation is completed / control device is working / control device or parts of the control device are not working); type of the device (water dispenser / water purifier); canister index; date of the last communication with the equipment; date of the last ozonation; date of installation, for example, of the equipment or the control device; ozonation program; operation program; controlled stop option; and component errors and settings.

The Android app G may connect to the equipment through the serial and may enable the initial configuration of the equipment, their association by client and location and the subsequent reading of communication errors (the ones that do not reach the server). The unique identification of the equipment may be performed through the IMEI. It is allocated a client and a location ID and the equipment type. Furthermore, the app allows the manual performance of some tests for verifying the following modules: GSM communication, air pump, green/redLED, display, ozone generator, electric valve, server communication.

The operational steps and/or the organizational chart of the program on which the operation of the invention is based are performed or executed or used by the microcontroller.

The microcontroller or an app in the microcontrollermay be in charge of managing the modem, such as starting and/or stopping the modem, communication through AT commands with the modem, parsing responses from the modem, and/or including unsolicited answers (URC - unsolicited result/response code).

Furthermore, the microcontroller or an app in the microcontroller may be in charge of RTCC management (clock), such as setting and/or retrieving the hour and date from the RTCC, RTCC configuration, and/or monitoring the date/hour at which the RTCC switches from the main supply to the back-up battery.

Furthermore, the microcontroller or an app in the microcontroller may be in charge of managing the switch for detecting canisters, such as for detecting the presence/absence of a canister on the dispenser, and counting the changed canisters; managing the ozonation process, such as the schedule for starting and/or stopping the ozone generator, pump, signaling through red LED or display;LCD management related to messages generated from the web interface, dynamic messages based on local actions (for example ozonation), other predefined messages; visual management related to text scrolling, backlight actions (starting, stopping, intermittent), the speed with which the text is moving, positioning, etc.; managing the water supply, such ascontrolling the electrical valve or solenoid valve; managing the supply of the hot and cold water dispensers or managing the water heater and cooler, such asscheduling by weekdays or setting the supply permanently on/off; managing firmware updates, such as an encrypted binary file received through GPRS, temporarily stored in the external EEPROM; managing the UART communication (serial), such as the communication used for the initial configuration of parameters, UART debug, test ozonation and for communication with the Android app.

### Second variant

A water cleaning system in the second variant, as illustrated in Fig. 2, comprises one or multiple water cleaning systems and a remote computing device S, wherein each of the water cleaning systems in the second variant comprises a water purifier 209, and wherein the water purifiers may be installed at different locations.

The water cleaning system in the second construction variant comprises a water cleaning device 15 and a water purifier 209.

The water cleaning system may further comprise a valve H, such as a solenoid valve H. The solenoid valve H has the purpose of controlling the water supply of water purifier-type devices, devices powered or supplied from the water network. The solenoid valve H may be controlled by the control device 14 based on data received from the remote computing device S. Usually, the solenoid valve may be closed. When it is intentionally closed from the web interface or when it is not electrically powered, it will be closed and will prevent the passing of water. During normal use, it will be open in order to allow the passing of water towards the device's tank. In order to save energy and diminish the self-heating effect, the electrical supply of the solenoid valve is performed through impulses. The rate of pulse filling used is variable (configurable). Periodically, solenoid valve H is applied a longer pulse, in order to ensure its open state. The solenoid valve H is supplied with 12 VDC direct current. It is made of nickel covered brass.

The configuration of the water cleaning device 15 and the configuration of other parts of the multiple-location water cleaning system in the second variant may be the same as or analogous to those in the first variant.

The operations of the multiple-location water cleaning system in the second variant and the operations of any parts of the multiple-location water cleaning system in the second variant may be the same as or analogous to those in the first variant.

### Operation

The equipments used may be the following, namely the ozonation equipment with display, GSM modem and canister counter; and the ozonating equipment with display, GSM modem and solenoid valve which generates ozone in the tank water.

The air pump and the ozone generator may be controlled independently by the control device, for example, based on first data and second data (which is different from the first data) received from the remote computing device.

The first data may comprise one or more of the following: a start time for the operation of the air pump 2, an end time for the operation of the air pump 2, a time interval for performing the operation of the air pump 2, a time interval for performing the operation of the air pump 2 after finishing an operation of the ozone generator 1, and a pump capacity of the air pump 2.

The second data may comprise one or more of the following: a start time for the operation of the ozone generator 1, an end time for the operation of the ozone generator 1, a time interval for performing the operation of the ozone generator 1, a voltage used in the ozone generator 1 to generate ozone from air, and a size of particles filtered by an air filter 1.4 in the ozone generator 1,

The first data may be based on the second data such that, for example, the air pump may be working for another 10 minutes (this time span is configurable) after the ozone generator has stopped.

The actual ozonation and an additional period of 10 minutes after its end (during which the air pump is working) may be marked through the lighting of a red LED or through a display which will display this information or warning, both of them being installed on the dispenser's housing in order to signal the fact that the water cannot be consumed during those 15 minutes.

The ozonation function may start and stop automatically at the hours established/set upon installation. The hour interval can be changed from the Management Solution. The Management Solution may be a remote computing device, such as a server, mobile terminal device, a user interface, a web interface, an app for a mobile computing device, or an Android app. The ozonation interval may take into account the day of the week.

The equipment may communicate to the server the fact that the ozonation was performed within the allocated interval. The web interface may display the date and hour when the last complete ozonation was finished.

If either the air pump or the ozone generator does not consume energy although they are powered, a specific message may be displayed in the Management solution.

The ozone generator and the electrical power of the dispenser (the water cooling part of the compressor, together with the heating part) can be scheduled independently of the starting/stopping hours. The schedule for the ozone generator can be set differently for each day of the week, and the electrical supply may be declared for each day of the week.

The diffusion stone 1.6 may be installed at the base of the tank (on the bottom of the tank) for maximum efficiency. The provisions of the User Manual may be followed, including those regarding the correct installation of the check valve 1.5, in order to avoid the strangulation of hoses.

### Counting of canisters

A water canister 8 or a plurality of water canisters 8 may be attached at, such as on or in, the water dispenser 109. The water canister 8 may store drinking water. Alternatively or in addition, the water dispenser may comprise a water tank, which may store drinking water. The water tank may be a water canister 8. Water may be exchanged between the water canister 8 and the water tank. The ozone-based water cleaning operation may be applied in the water tank and/or in the water canister 8. A user of the water dispenser 109 may receive drinking water from the water tank and/or from the water canister 8. The water canister 8 may be replaced/changed with another water canister 8. In particular, the water canister 8 may be replaced/changed when a water canister 8 is empty.

When changing a canister 8 with another canister 8, the switch may register the change and may increase the index by 1 and may communicate the new index to the Management Solution immediately after completing the action. The interface may display the number of canisters 8 changed (total number since the installation of the equipment or for a certain time interval selected in the interface) and the date of the last canister change.

Periodically, a status with the presence of a canister 8 detected by the switch on the dispenser 109 may be sent: canister present/absent. This information may be visible in the Management Solution through a red / green button.

The equipment may store, in a non-volatile memory, the index of changed canisters. Therefore, the index will not be lost due to blackouts.

By counting the number of canisters changed, for example, a provider or seller of the control device may determine the max. amount of water to which the ozone cleaning operation was applied (like an upper threshold); the amount of water to which the ozone cleaning is applied as a function of time, such as a function of the weekday; the amount of time left before parts of the water cleaning system must be cleaned, renewed or replaced; and/or the debt of a client or buyer of the control device.

### Communication and other functions

The communication between the equipment and the Management Solution may be performed with the help of mobile communication, using a modem and a data subscription corresponding to a SIM card.

Optionally, equipment needs to be installed by the Beneficiary/Clients/collaborators/third parties authorized by the Beneficiary, on the dispensers, and the information connecting the client's information to the equipment, that has a unique ID, need to be declared.

The equipment may have an internal clock (a battery for the management of the date/hour) based on which it decides the beginning and end of the ozonation period and the scheduled stop for reducing energy consumption.

There may in particular be 2 functions for stopping the dispenser's energy supply: the interruption of energy supply is desired in order to reduce energy consumption or is desired permanently due to non-payment.

### Display

The solution enables the subsequent installation of a display, integrated in a housing that will be installed on the dispenser's/purifier's exterior. It will be connected with the equipment using a cable that allows the fast and easy dismantling of the Display from the dispenser / purifier. The information displayed on such Displays can be generated locally or can be transmitted from the Server through the Management Solution (Web or Android) using a GSM communication. Messages can be generated by default, can be automatically generated or can be manually drafted.

### The present invention may alternatively be described by the following numbered aspects of the invention:

1. A control device (14) for a liquid dispenser (109) or liquid purifier (209), the control device (14) comprising: a communication unit (3) configured to receive data from a remote computing device (S), and a control unit (12) configured to control an ozone-based liquid cleaning operation in the liquid dispenser (109) or liquid purifier (209) based on the received data.
2. A control device (14) according to aspect 1, further comprising an internal clock (4), wherein controlling the liquid cleaning operation is further based on a time provided by the internal clock (4), and wherein the internal clock (4) is preferably equipped with an internal backup battery (11).
3. A control device (14) according to any one of aspects 1 and 2, wherein the received data comprises at least one of a start time for the liquid cleaning operation, an end time for the liquid cleaning operation, and a time interval for performing the liquid cleaning operation.
4. A control device (14) according to any one of aspects 1 to 3, wherein the communication unit (3) is further configured to send data to the remote computing device (S), wherein the data sent to the remote computing device (S) comprises at least one of identity information of the control device (14), identity information of a user of the control device (14), location information of the liquid dispenser (109) or liquid purifier (209), type information of the liquid dispenser (109) or liquid purifier (209), status information of the control device (14), information about a current status of the liquid cleaning operation, and status information of the liquid dispenser (109) or liquid purifier (209).
5. A control device (14) according to aspect 4, wherein the data sent to the remote computing device (S) is for output in a user interface at the remote computing device (S), and wherein the data received from the remote computing device (S) is based on the data sent to the remote computing device (S) and/or on a user input in the user interface, and wherein the user interface comprises at least one of a web interface (F) and an app (G) for a mobile computing device.
6. A control device (14) according to any one of aspects 1 to 5, further comprising a display (7) configured to display a current status of the liquid cleaning operation when the liquid cleaning operation is being performed in the liquid dispenser (109) or liquid purifier (209).
7. A control device 14 according to any one of aspects 1 to 6, further comprising a memory, wherein the control device 14 is configured to store the received data in the memory.
8. A control device 14 according to any one of aspects 1 to 7, further comprising hardware and/or software protections configured to prevent an overcurrent and/or to limit a time interval for performing the liquid cleaning operation and/or to protect data received from the remote computing device S and/or to protect data sent to the remote computing device S.
9. A liquid cleaning device (15) comprising a control device (14) according to any one of aspects 1 to 8 and a liquid cleaning unit (A) configured to perform the liquid cleaning operation.
10. A liquid cleaning device (15) according to aspect 9, wherein the liquid cleaning unit (A) comprises an air pump (2) and an ozone generator (1), wherein the air pump (2) is configured to receive air from the atmosphere and to pump the received air into the ozone generator (1), and wherein the ozone generator (1) is configured to generate ozone based on the received air and to release the generated ozone into liquid of the liquid dispenser (109) or liquid purifier (209).
11. A liquid cleaning device (15) according to aspect 10, wherein the control unit (12) of the control device (14) is further configured to control an operation of the air pump (2) based on first data received from the remote computing device (S) and to control an operation of the ozone generator (1) based on second data received from the remote computing device (S).
12. A liquid cleaning device (15) according to aspect 11, wherein the first data comprises at least one of a start time for the operation of the air pump (2), an end time for the operation of the air pump (2), a time interval for performing the operation of the air pump (2), a time interval for performing the operation of the air pump (2) after finishing an operation of the ozone generator (1), and a pump capacity of the air pump (2), and wherein the second data comprises at least one of a start time for the operation of the ozone generator (1), an end time for the operation of the ozone generator (1), a time interval for performing the operation of the ozone generator (1), a voltage used in the ozone generator (1) to generate ozone from air, and a size of particles filtered by an air filter (1.4) in the ozone generator (1), and wherein the first data is preferably based on the second data.
13. A liquid cleaning system comprising a liquid dispenser (109); a liquid cleaning device (15) according to any one of aspects 9 to 12; and a switch (6) configured to detect the presence or absence of a liquid canister (8) on the liquid dispenser (109) and/or to count the number of liquid canisters (8) changed on the liquid dispenser (109).
14. A liquid cleaning system according to aspect 13, wherein the control device (14) is a control device (14) according to any one of aspects 4 to 8, and wherein the data sent to the remote computing device (S) further comprises at least one of the presence or absence of a liquid canister (8) on the liquid dispenser (109), the number of liquid canisters (8) changed on the liquid dispenser, and the time of the last canister change.
15. A liquid cleaning system according to any one of aspects 13 and 14, wherein the liquid dispenser (109) comprises a liquid cooler and heater, and wherein the control device (14) is further configured to output an electrical signal to control the liquid cooler and heater based on data received from the remote computing device (S).
16. A liquid cleaning system comprising a liquid purifier (209), wherein the liquid purifier (209) comprises a liquid supply; a liquid cleaning device (15) according to any one of aspects 9 to 12; and a valve (H) configured to control the liquid supply, wherein the control device (14) is further configured to control the valve (H) based on data received from the remote computing device (S).
17. A multiple-location liquid cleaning system comprising a plurality of liquid cleaning systems according to any one of aspects 13 to 16 and the remote computing device (S), wherein the plurality of liquid dispensers (109) or liquid purifiers (209) are installed at different locations.
18. A computer-implemented control method for a liquid dispenser (109) or liquid purifier (209), the method comprising: receiving data from a remote computing device (S), and controlling an ozone-based liquid cleaning operation in the liquid dispenser (109) or liquid purifier (209) based on the received data.

### The present invention may alternatively also be described by the following

### numbered aspects of the invention:

1a.The equipment for the control of the water ozonation and management and communication with water dispensers and purifiers installed in various locations, **characterized through the fact that** it is used in or for water dispensers, being composed of an ozonator (A) composed of an ozone generator (1) and an air pump (2), wherein the air pump retrieves air from the atmosphere and pumps it through a tube into the ozone generator (1), a switch (6) with the purpose of counting canisters, which is installed within the dispenser (9) and positioned with the actuator on the contact surface between the canister (8) and the dispenser, a GSM communication system (3), an internal clock (4) equipped with an internal backup battery (11), hardware and software protections (5), a server (S), a web interface (F) and an Android app (G), wherein some or all of these components are connected with or controlled by or based on a microcontroller (12), and wherein the equipment is used in of for water dispensers.
2a.The equipment compliant to aspect 1a, **characterized through the fact that** the ozone generator (1) has an enclosure (1.1) connected to inlet tube (1.2) and an outlet tube (1.3), an air filter (1.4), which has the purpose of preventing particles preferably larger than 0.45 micrometers present in the air from getting into the water tank, a check valve (1.5) which prevents water in the basin or tank from getting into the ozone generator 1, and in order to maximize the bubble generating surface, it uses a spherical diffusion stone (1.6).
3a.The equipment compliant to aspect 1a, **characterized by the fact that** the switch (6) is an electromechanical switch.
4a.The equipment compliant to aspect 1a, **characterized by the fact that** the GSM communication system (3) contains a GSM/GPRS (2G), quad-band modem, which uses a SIM card 13 for a bidirectional data exchange with the server (S).
5a.The equipment compliant to aspect 1a, **characterized by the fact that** the hardware and software protections (5) are arranged in a housing (10) and consist of a safety fuse for overcurrent, a WDT surveillance chronometer for unexpected SW situations, a timed switch for limiting the maximum ozonation duration, checksum for protecting the data stored and the data sent through GSM/GPRS.
6a.The equipment for the control of water ozonation and management and communication with water purifiers installed in different locations, **characterized by the fact that** it is composed of an ozonator (A), consisting of an ozone generator (1), a solenoid valve (H), a GSM communication system (3), an internal clock (4), hardware and software protections (5), a server (S), a web interface (F) and an Android app (G).

The above examples, variants, aspects and embodiments are preferred realizations of the present invention, wherein the present invention is defined by the following appended claims.

## Claims

1. A control device (14) for a liquid dispenser (109) or liquid purifier (209), the control device (14) comprising:
a communication unit (3) configured to receive data from a remote computing device (S), and
a control unit (12) configured to control an ozone-based liquid cleaning operation in the liquid dispenser (109) or liquid purifier (209) based on the received data.

2. A control device (14) according to claim 1, further comprising
an internal clock (4),
wherein controlling the liquid cleaning operation is further based on a time provided by the internal clock (4),
and wherein the internal clock (4) is preferably equipped with an internal backup battery (11).

3. A control device (14) according to any one of claims 1 and 2,
wherein the received data comprises at least one of a start time for the liquid cleaning operation, an end time for the liquid cleaning operation, and a time interval for performing the liquid cleaning operation.

4. A control device (14) according to any one of claims 1 to 3,
wherein the communication unit (3) is further configured to send data to the remote computing device (S), wherein the data sent to the remote computing device (S) comprises at least one of identity information of the control device (14), identity information of a user of the control device (14), location information of the liquid dispenser (109) or liquid purifier (209), type information of the liquid dispenser (109) or liquid purifier (209), status information of the control device (14), information about a current status of the liquid cleaning operation, and status information of the liquid dispenser (109) or liquid purifier (209).

5. A control device (14) according to claim 4,
wherein the data sent to the remote computing device (S) is for output in a user interface at the remote computing device (S),
and wherein the data received from the remote computing device (S) is based on the data sent to the remote computing device (S) and/or on a user input in the user interface,
and wherein the user interface comprises at least one of a web interface (F) and an app (G) for a mobile computing device.

6. A control device (14) according to any one of claims 1 to 5, further comprising
a display (7) configured to display a current status of the liquid cleaning operation when the liquid cleaning operation is being performed in the liquid dispenser (109) or liquid purifier (209).

7. A liquid cleaning device (15) comprising a control device (14) according to any one of claims 1 to 6 and a liquid cleaning unit (A) configured to perform the liquid cleaning operation.

8. A liquid cleaning device (15) according to claim 7,
wherein the liquid cleaning unit (A) comprises an air pump (2) and an ozone generator (1),
wherein the air pump (2) is configured to receive air from the atmosphere and to pump the received air into the ozone generator (1),
and wherein the ozone generator (1) is configured to generate ozone based on the received air and to release the generated ozone into liquid of the liquid dispenser (109) or liquid purifier (209).

9. A liquid cleaning device (15) according to claim 8,
wherein the control unit (12) of the control device (14) is further configured to control an operation of the air pump (2) based on first data received from the remote computing device (S) and to control an operation of the ozone generator (1) based on second data received from the remote computing device (S).

10. A liquid cleaning system comprising
a liquid dispenser (109);
a liquid cleaning device (15) according to any one of claims 7 to 9; and
a switch (6) configured to detect the presence or absence of a liquid canister (8) on the liquid dispenser (109) and/or to count the number of liquid canisters (8) changed on the liquid dispenser (109).

11. A liquid cleaning system according to claim 10,
wherein the control device (14) is a control device (14) according to any one of claims 4 to 6,
and wherein the data sent to the remote computing device (S) further comprises at least one of the presence or absence of a liquid canister (8) on the liquid dispenser (109), the number of liquid canisters (8) changed on the liquid dispenser, and the time of the last canister change.

12. A liquid cleaning system according to any one of claims 10 and 11,
wherein the liquid dispenser (109) comprises a liquid cooler and heater,
and wherein the control device (14) is further configured to output an electrical signal to control the liquid cooler and heater based on data received from the remote computing device (S).

13. A liquid cleaning system comprising
a liquid purifier (209), wherein the liquid purifier (209) comprises a liquid supply;
a liquid cleaning device (15) according to any one of claims 7 to 9;
and a valve (H) configured to control the liquid supply,
wherein the control device (14) is further configured to control the valve (H) based on data received from the remote computing device (S).

14. A multiple-location liquid cleaning system comprising a plurality of liquid cleaning systems according to any one of claims 10 to 13 and the remote computing device (S), wherein the plurality of liquid dispensers (109) or liquid purifiers (209) are installed at different locations.

15. A computer-implemented control method for a liquid dispenser (109) or liquid purifier (209), the method comprising:
receiving data from a remote computing device (S), and
controlling an ozone-based liquid cleaning operation in the liquid dispenser (109) or liquid purifier (209) based on the received data.
